# EUROPEAN PATENT SPECIFICATION

(11) **EP 3 259 260 B1**
(45) Date of publication and mention of the grant of the patent: **12.12.2018**
(21) Application number: 16705141.6
(22) Date of filing: 17.02.2016
(51) Int. Cl.: C07D 301/12

(54) **METHOD FOR THE EPOXIDATION OF AN OLEFIN WITH HYDROGEN PEROXIDE**
VERFAHREN ZUR EPOXIDIERUNG EINES OLEFINS MIT WASSERSTOFFPEROXID
PROCÉDÉ D'ÉPOXYDATION D'UNE OLÉFINE AVEC DU PEROXYDE D'HYDROGÈNE

(30) Priority: 17.02.2015 EP 15155400
(43) Date of publication of application: 27.12.2017
(73) Proprietor: Evonik Degussa GmbH, 45128 Essen (DE)
(72) Inventor: BREITENBACH, Uwe, 63584 Gründau (DE); IMM, Sebastian, 61118 Bad Vilbel (DE); RAUSCH, Hans-Martin, 65760 Eschborn (DE); PASCALY, Matthias, 60599 Frankfurt (DE); STOCK, Jürgen, 60599 Frankfurt (DE)
(86) International application number: PCT/EP2016/053340
(87) International publication number: WO 2016/131858

(56) References cited:
- WO-A1-2004/043941
- WO-A1-2011/063937
- US-A1- 2002 004 606

## Description

### Field of the invention

The invention relates to a method for the epoxidation of an olefin with hydrogen peroxide in the presence of a homogeneous epoxidation catalyst where the reaction is carried out in a reaction mixture comprising an aqueous liquid phase and an organic liquid phase.

### Background of the invention

Methods for the epoxidation of an olefin with hydrogen peroxide using a homogeneous epoxidation catalyst are known from the prior art.

Epoxidation of an olefin with hydrogen peroxide using a water soluble manganese complex as epoxidation catalyst is known from D. E. De Vos et al., Tetrahedron Letters 39 (1998) 3221-3224 and from US 5,329,024. WO 2010/012361 teaches to carry out the epoxidation in a biphasic system comprising an organic phase and an aqueous phase. The pH of the reaction medium is preferably stabilized in the range of from 3.7 to 4.2 using a buffer of oxalic acid and sodium oxalate.

WO 2011/107188 discloses epoxidation of olefins with hydrogen peroxide in the presence of a water soluble manganese complex comprising a 1,4,7-trimethyl-1,4,7-triazacyclonane ligand. Epoxidation is carried out in a loop reactor in a multiphasic reaction mixture comprising an organic phase and an aqueous phase. The pH of the aqueous phase is stabilized in the range of from 2 to 5 using a buffer system.

US 5,274,140 discloses epoxidation of olefins with hydrogen peroxide in the presence of a catalyst system comprising a heteropolytungstate as epoxidation catalyst and a quaternary ammonium or phosphonium salt as phase transfer catalyst. The pH of the aqueous phase can be in the range of from 2 to 6.

For an epoxidation in a two phase mixture, where most of the olefin is in the organic phase and most of the hydrogen peroxide is in the aqueous phase, mixing the liquid phases to provide a large area of the phase boundary improves mass transfer between the phases and increases reaction rates of epoxidation.

### Summary of the invention

It has been found that for epoxidation of an olefin with hydrogen peroxide in a two phase mixture carrying out the epoxidation continuously in a loop reactor and controlling the pH of the aqueous phase within a narrow range allows to achieve both high epoxide selectivity and low decomposition of hydrogen peroxide to oxygen.

It has also been found that in a well-mixed reaction mixture pH measurement with a pH electrode is unreliable and often gives erroneous results for extended time periods. The use of such an erroneous result for controlling pH during an epoxidation reaction will lead to maladjustment of pH with the result of either lowered epoxide selectivity or increased hydrogen peroxide decomposition. Withdrawing samples and determining pH on the withdrawn sample after phase separation does not solve this problem, because the pH rapidly changes in samples withdrawn from the reaction mixture.

It has further been found that in an epoxidation of an olefin with hydrogen peroxide in a two phase mixture the pH of the aqueous phase can be determined reliably, even when operating with strong mixing of the two liquid phases, by arranging a pH electrode in a measuring section where the liquid phases of the reaction mixture are temporarily separated and the pH electrode is in contact with the separated aqueous phase.

Subject of the invention is therefore a method for the epoxidation of an olefin comprising continuously reacting the olefin with hydrogen peroxide in the presence of a homogeneous epoxidation catalyst. The reaction is carried out in a reaction mixture comprising an aqueous liquid phase and an organic liquid phase using a loop reactor with mixing of the liquid phases. The loop reactor comprises a measuring section in which the liquid phases are temporarily separated into a separated aqueous phase and a separated organic phase and at least one pH electrode is arranged in said measuring section in contact with the separated aqueous phase. The liquid phases are preferably separated by lowering the flow rate. A pH of the separated aqueous phase is determined with the pH electrode and this pH is maintained in a predetermined range by adding acid or base to the loop reactor.

### Brief description of drawings

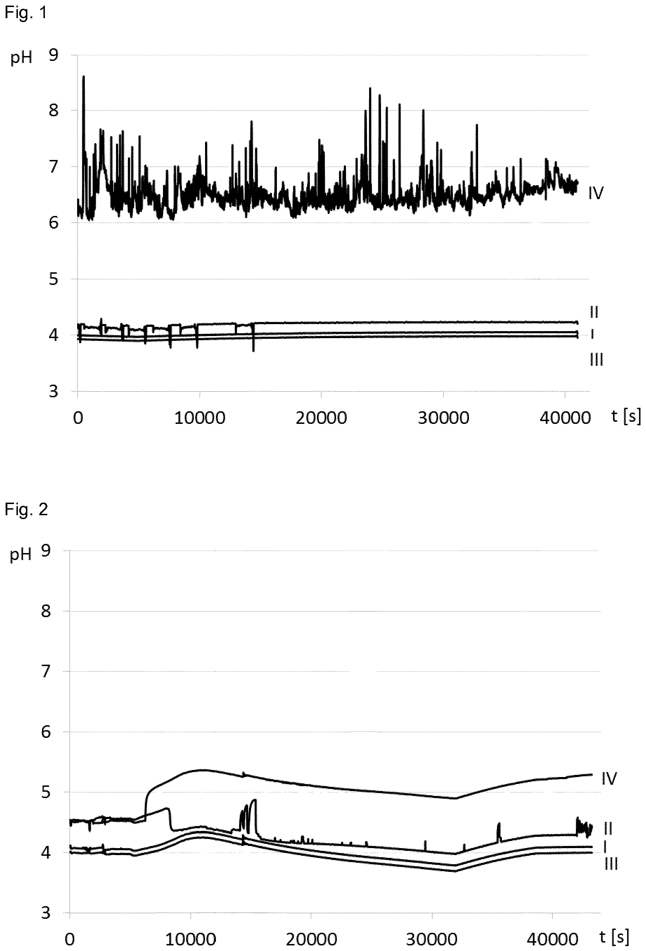

Fig. 1 and 2 show pH measurements for epoxidation of propene in a loop reactor with pH electrodes I, II and III arranged in a measuring section with temporary phase separation in the measuring section and pH electrode IV arranged in the mixed main flow of the loop reactor.

### Detailed description of the invention

In the method of the invention an olefin is reacted with hydrogen peroxide in the presence of a homogeneous epoxidation catalyst in a reaction mixture comprising an aqueous liquid phase and an organic liquid phase.

The olefin may contain one or several carbon-carbon double bonds. In olefins containing two or more double bonds, the double bonds may be isolated or conjugated, isolated double bonds being preferred. The olefin may be linear, branched or cyclic and may carry substituents, in particular one or more substituents selected from aryl groups, halogens, free and esterified hydroxyl groups, alkoxy groups and carboxyl groups. The substituents may be in vinylic or allylic position or bonded to another position of the olefin, with substituents in allylic position being preferred.

In a preferred embodiment, the olefin is allyl chloride and the method of the invention provides epichlorohydrin as the reaction product. In another preferred embodiment, the olefin is propene and the method of the invention provides propene oxide as the reaction product.

Hydrogen peroxide can be used as an aqueous solution, preferably containing from 20 to 75 % by weight hydrogen peroxide and most preferably from 40 to 70 % by weight. Preferably, an aqueous hydrogen peroxide solution prepared by an anthraquinone process is used. A crude hydrogen peroxide solution as obtained in the extraction step of the anthraquinone process may be used in the method of the invention.

In one embodiment of the invention, the homogeneous epoxidation catalyst is a water soluble epoxidation catalyst comprising a manganese complex. The manganese complex preferably comprises at least one polydentate ligand which preferably coordinates through nitrogen atoms, most preferably through tertiary amino groups. The manganese complex may be a mononuclear complex of formula [LMnXₘ]Yₙ, a dinuclear complex of formula [LMn(µ-X)ₘMnL]Yₙ or a polynuclear complex of formula [LₚMnₚ(µ-X)ₘ]Yₙ, where L is a polydentate ligand, X is a coordinating species, µ-X is a bridging coordinating species, Y is a non-coordinating counter ion, m is 1, 2 or 3, n is an integer providing for the charge neutrality of the complex, and p is from 3 to 5. X and µ-X are preferably selected from the group consisting of RO⁻, Cl⁻, Br⁻, I⁻, F⁻, NCS⁻, N₃⁻, I₃⁻, NH₃, NR₃, RCOO⁻, RSO₃⁻, ROSO₃⁻, OH⁻, O²⁻, O₂²⁻, HOO⁻, H₂O, SH⁻, CN⁻, OCN⁻, C₂O₄²⁻ and SO₄²⁻, where R is alkyl, cycloalkyl, aryl or aralkyl with no more than 20 carbon atoms. Y is preferably selected from the group consisting of RO⁻, Cl⁻, Br⁻, I⁻, F⁻, RCOO-, SO₄²⁻, PF₆⁻, p-tolylsulfonate and trifluoromethylsulfonate, where R is alkyl, cycloalkyl, aryl or aralkyl with no more than 20 carbon atoms. Manganese may be in the oxidation state +2, +3, +4, or +7, the oxidation states +3 and +4 being preferred.

Preferred polydentate ligands are acyclic polyamines containing at least 7 atoms in the backbone or cyclic polyamines containing at least 9 atoms in the ring, each having the nitrogen atoms separated by at least two carbon atoms. Most preferred are ligands having a 1,4,7-triazacyclononane (Tacn) ring system, which may be substituted with one or more alkyl, cycloalkyl, aryl or aralkyl groups each containing up to 20 carbon atoms. Preferred substituents are methyl groups. Suitable ligands with a Tacn ring system are N',N",N"'-trimethyl-1,4,7-triazacyclononane (TmTacn) and 2-methyl-1,4,7-trimethyl-1,4,7-triazacyclononane, with TmTacn being preferred. Another suitable ligand is 1,5,9-trimethyl-1,5,9-triazacyclododecane.

Most preferred are the dinuclear manganese complexes [(TmTacn)Mn^{IV}(µ-O)₃Mn^{IV}(TmTacn)](PF₆)₂ and [(TmTacn)Mn^{IV}(µ-O)₃Mn^{IV}(TmTacn)](CH₃COO)₂.

The manganese complex may be formed in the reaction mixture by reaction of the polydentate ligand with a manganese salt, preferably manganese sulfate, manganese acetate, manganese nitrate, manganese chloride or manganese bromide with Mn²⁺ or Mn³⁺. Preferably, the manganese complex is prepared separately and added to the reaction mixture.

The water soluble epoxidation catalyst preferably comprises oxalic acid, an oxalate or a mixture of both as a co-catalyst in addition to the manganese complex. The co-catalyst is preferably used in a molar excess to the manganese complex, preferably with a molar ratio of co-catalyst to manganese complex in the range of from 10 : 1 to 10 000 : 1.

When a water soluble epoxidation catalyst is used, the olefin preferably has a solubility in water of from 0.01 g/L to 100 g/L at 20°C, more preferably of from 0.01 g/L to 10 g/L at 20°C, in order to achieve both a high rate of reaction in epoxidation and formation of an organic liquid phase without addition of solvent. The organic phase may contain a water insoluble solvent, but preferably contains less than 30 % by weight, more preferably less than 5 % by weight of a solvent.

In another embodiment of the invention, the homogeneous epoxidation catalyst comprises a heteropolytungstate. The heteropolytungstate preferably comprises phosphorus or arsenic as heteroatom, most preferably phosphorus, i.e. the heteropolytungstate is a polytungstophosphate. Heteropolytungstates are known from the prior art. Most preferred are polytungstophosphates with a molar ratio of phosphorus to tungsten of from 1 : 2 to 1 : 12. The polytungstophosphate is preferably generated in situ from phosphoric acid and sodium tungstate, which are preferably employed in a molar ratio of phosphorus to tungsten of from 1 : 2 to 10 : 1. A polytungstophosphate reacts with hydrogen peroxide in the aqueous phase to give peroxotungstates and peroxotungstophosphates, such as PO₄[WO(O₂)₂]₄³⁻ and HPO₄[WO(O₂)₂]₂²⁻ and the corresponding partially protonated species.

The heteropolytungstate is preferably used in combination with a phase transfer catalyst. The term phase transfer catalyst refers to a compound comprising a cation or forming a cation in the aqueous phase, which cation forms a salt with the peroxotungstate and peroxotungstophosphate that is soluble in the organic phase. The phase transfer catalyst preferably comprises a singly charged cation or a compound forming a singly charged cation in the aqueous phase. Suitable as phase transfer catalysts are quaternary ammonium salts, tertiary amines and quaternary phosphonium salts. Suitable quaternary ammonium salts are tetraalkylammonium salts comprising at least 12 carbon atoms in total in the alkyl groups, such as dodecyltrimethylammonium salts, hexadecyltrimethylammonium salts, octadecyltrimethylammonium salts, methyltributylammonium salts and methyltrioctylammonium salts. Suitable quaternary ammonium salts may comprise singly and doubly charged anions, for example chloride, bromide, nitrate, sulfate, hydrogenphosphate, dihydrogenphosphate, methylsulfonate, methylsulfate and ethylsulfate. Suitable tertiary amines are dodecyldimethylamine, hexadecyldimethylamine, octadecyldimethylamine, tributylamine and trioctylamine.

The phase transfer catalyst is preferably used in an amount providing a molar ratio of phase transfer catalyst to tungsten of from 0.2 : 1 to 3 : 1, more preferably from 0.4 : 1 to 1 : 1, the molar ratio referring to the amount of cations or cation forming compounds in the phase transfer catalyst.

Preferably, the phase transfer catalyst comprises a salt with a quaternary ammonium ion of structure R¹R²R³R⁴N⁺, where R¹ is a group Y-O(C=O)R⁵, Y being a group CH₂CH₂, CH(CH₃)CH₂ or CH₂CH(CH₃) and R⁵ being an alkyl or alkenyl group containing 11 to 21 carbon atoms, R² is an alkyl group containing 1 to 4 carbon atoms and R³ and R⁴ are, independently of one another, R¹, R² or Y-OH. Preferred are salts with methyl sulfate as anion, for which R² is methyl and R⁵ is a linear alkyl or alkenyl group. More preferred are the salts (CH₃)₃N⁺CH₂CH₂O(C=O)R⁵ CH₃OSO₃⁻, (CH₃)₂N⁺(CH₂CH₂OH)(CH₂CH₂O(C=O)R⁵) CH₃OSO₃⁻, (CH₃)₂N⁺(CH₂CH₂O(C=O)R⁵)₂ CH₃OSO₃⁻, CH₃N⁺(CH₂CH₂OH)₂(CH₂CH₂O(C=O)R⁵) CH₃OSO₃⁻, CH₃N⁺(CH₂CH₂OH)(CH₂CH₂O(C=O)R⁵)₂ CH₃OSO₃⁻, CH₃N⁺(CH₂CH₂O(C=O)R⁵)₃ CH₃OSO₃⁻, (CH₃)₃N⁺CH₂CH(CH₃)O(C=O)R⁵ CH₃OSO₃⁻, (CH₃)₂N⁺(CH₂CH(CH₃)OH)(CH₂CH(CH₃)O(C=O)R⁵) CH₃OSO₃⁻ and (CH₃)₂N⁺(CH₂CH(CH₃)O(C=O)R⁵)₂ CH₃OSO₃⁻, in which R⁵ is a linear alkyl or alkenyl group containing 11 to 21 carbon atoms. Most preferred is the salt (CH₃)₂N⁺(CH₂CH(CH₃)O(C=O)R⁵)₂ CH₃OSO₃⁻ in which R⁵ is a linear alkyl or alkenyl group containing 11 to 17 carbon atoms. These preferred phase transfer catalysts can be prepared by esterifying ethanolamine, isopropanolamine, diethanolamine, diisopropanolamine, triethanolamine or triisopropanolamine with a fatty acid followed by quaternization with dimethylsulfate. Compared to tetraalkylammonium salts, these phase transfer catalysts have the advantage of being readily biodegradable which allows direct discharge of aqueous effluents from the method of the invention to a biological waste water treatment. Compared to tetraalkylammonium halides, these phase transfer catalysts provide reaction mixtures that are less corrosive. These preferred phase transfer catalysts are preferably added as a mixture comprising from 5 to 50 % by weight of a solvent selected from ethanol, 2-propanol, aliphatic hydrocarbons, fatty acids and fatty acid triglycerides to facilitate dosing and dispersing in the reaction mixture.

The heteropolytungstate and the phase transfer catalysts may be added as a mixture or separately, with separate addition to the reaction mixture being preferred.

When a heteropolytungstate is used in combination with a phase transfer catalysts, the resulting homogeneous epoxidation catalyst will be largely present in the organic liquid phase of the reaction mixture. With such a homogeneous epoxidation catalyst the epoxidation can be carried out both with and without addition of a solvent, which is preferably not water miscible. Preferably, an epoxidized fatty acid methyl ester is used as solvent. As an alternative to adding an epoxidized fatty acid methyl ester to the reaction mixture, the methyl ester of the corresponding unsaturated fatty acid may be added, which is then converted in the reaction mixture to the epoxidized fatty acid methyl ester. Preferred are epoxidized fatty acid methyl ester containing fatty acids derived from vegetable oils, preferably soy oil. Addition of an epoxidized fatty acid methyl ester prevents the formation of stable emulsions and facilitates phase separation of the two liquid phases of the reaction mixture. The solvent is preferably added in an amount providing a solvent content of the organic liquid phase of the reaction mixture of from 10 to 90 % by weight.

The reaction is carried out in a reaction mixture comprising an aqueous liquid phase and an organic liquid phase with mixing of the liquid phases. Preferably, the ratio of the volume of the aqueous phase to the volume of the organic phase is maintained in the range of from 10 : 1 to 1 : 10. When an epoxidation catalyst comprising a manganese complex is used, the ratio is more preferably from 2 : 1 to 1 : 4. Mixing of the liquid phases can be performed by turbulent flow of the reaction mixture, by passing reaction mixture through fixed mixing elements, such as static mixers, structured packings or random packings, or by a moving mixing element, such as a stirrer or a rotating pump.

Independently of which type of homogeneous epoxidation catalyst is used, the aqueous phase preferably comprises less than 30 % by weight, more preferably less than 5 % by weight of a solvent. The term solvent here refers to compounds added in addition to olefin, epoxidation catalyst, co-catalyst or phase transfer catalyst, and impurities introduced with these components, and does not encompass products formed from the olefin.

When an epoxidation catalyst comprising a manganese complex is used, the epoxidation reaction is preferably carried out at a temperature of from 0 °C to 70 °C, more preferably from 5 °C to 40 °C and most preferably from 10 °C to 30 °C. When an epoxidation catalyst comprising a heteropolytungstate is used, the epoxidation reaction is preferably carried out at a temperature of from 30 °C to 100 °C, more preferably from 60 °C to 90 °C.

When the boiling point of the olefin at 1 bar is close to or higher than the reaction temperature, the epoxidation is carried out at elevated pressure to maintain the olefin in the liquid phase.

The reaction is carried out continuously in a loop reactor. The term loop reactor here refers to a reactor in which reaction mixture is circulated driven by a pump. Pumping of the reaction mixture provides mixing of the liquid phases. The loop reactor may comprise vessels for increasing the volume in the loop and providing the residence time necessary for achieving the desired hydrogen peroxide conversion. Preferably, further mixing of the reaction mixture is provided in such vessels, for example by static mixers, structured packings or random packings arranged in a tube of enlarged diameter or by a stirred vessel arranged in the reactor loop. Preferably, a heat exchanger is arranged in the loop for cooling the reaction mixture in order to remove the heat of reaction, the reaction mixture preferably being passed through the heat exchanger in every cycle of the loop. The heat exchanger is preferably a tube bundle heat exchanger with the reaction mixture being passed through the tubes or a plate heat exchanger. The diameter of the tubes or the distance between plates is preferably chosen sufficiently narrow for providing turbulent flow and mixing of the two liquid phases.

The average residence time in the loop reactor, calculated as the ratio of the volume of the loop reactor divided by the sum of all fluid flows entering the loop reactor, is preferably selected to provide a hydrogen peroxide conversion of more than 85 %, more preferably of from 95 % to 99.5 %. For this purpose, the average residence time is preferably from 20 to 240 min.

The olefin is preferably used in molar excess to hydrogen peroxide in order to achieve high conversion of hydrogen peroxide and the molar ratio of olefin fed to the loop reactor to hydrogen peroxide fed to the loop reactor is preferably from 1.2 : 1 to 12 : 1, more preferably from 2 : 1 to 8 : 1.

When an epoxidation catalyst comprising a manganese complex is used, the amount of catalyst fed to the loop reactor is preferably chosen to provide a molar ratio of hydrogen peroxide fed to the loop reactor to manganese fed to the loop reactor of from 100 : 1 to 10 000 000 : 1, more preferably from 1000 : 1 to 1 000 000 : 1 and most preferably 10 000 : 1 to 100 000 : 1. When an epoxidation catalyst comprising a heteropolytungstate is used, the amount of catalyst fed to the loop reactor is preferably chosen to provide a molar ratio of hydrogen peroxide fed to the loop reactor to tungsten fed to the loop reactor of from 10 : 1 to 10 000 : 1, more preferably from 50 : 1 to 5 000 : 1.

When an epoxidation catalyst comprising a manganese complex is used, the concentration of hydrogen peroxide in the aqueous liquid phase is preferably maintained at less than 1.0 % by weight during the reaction. More preferably, the concentration of hydrogen peroxide is maintained at from 0.1 to 1.0 % by weight, most preferably at from 0.2 to 0.7 % by weight. When an epoxidation catalyst comprising a heteropolytungstate is used, the concentration of hydrogen peroxide in the aqueous liquid phase is preferably maintained at from 0.1 to 5 % by weight, preferably 0.5 to 3 % by weight. The concentration of hydrogen peroxide in the aqueous liquid phase may be adjusted by adjusting the molar ratio of olefin to hydrogen peroxide fed to the loop reactor, adjusting the feed rate for feeding hydrogen peroxide to the loop reactor or adjusting the feed rate for feeding epoxidation catalyst to the reactor, with a higher molar ratio of olefin to hydrogen peroxide, a lower feed rate for hydrogen peroxide or a higher feed rate for epoxidation catalyst leading to a lower concentration of hydrogen peroxide in the aqueous liquid phase.

The method of the invention uses a loop reactor comprising a measuring section, in which the liquid phases are temporarily separated into a separated aqueous phase and a separated organic phase. At least one pH electrode is arranged in the measuring section in contact with the separated aqueous phase, and a pH of the separated aqueous phase is determined with the pH electrode.

The measuring section may be located in the main loop of the loop reactor, but is preferably located in a side stream to the loop reactor. The term side stream here refers to a stream which is continuously withdrawn from the loop reactor and is at least partially returned to the loop reactor. Preferably, the entire side stream is returned to the loop reactor. Flow rate in the side stream may be adjusted independently of the flow rate in the main loop, for example by a pump or by a valve in the side stream.

The liquid phases can be temporarily separated by settling or by centrifugal force and are preferably separated by lowering the flow rate which leads to settling. In a preferred embodiment, the flow rate is lowered in the measuring section by enlarging the flow cross section. Preferably, a side stream is passed through a horizontal pipe having a section with an enlarged diameter where lowering of the flow rate leads to temporary phase separation by settling. In another preferred embodiment, the measuring section is located in a side stream and a valve is used for lowering the flow rate or temporarily stopping the flow in the measuring section. Preferably, the flow rate is only reduced but not entirely stopped.

In principle, any pH electrode known from the prior art, known to be suitable for measuring pH in the presence of organic compounds and stable to hydrogen peroxide can be used in the method of the invention. Preferably, a glass electrode is used as pH electrode, more preferably a combination electrode, which combines both the glass and reference electrode into one body.

Reliability of the pH detection can be improved by measuring pH with more than one pH electrode, preferably using at least three pH electrodes and most preferably using three or four pH electrodes. The pH electrodes are then preferably arranged side by side in the measuring section, more preferably with minimum distance between the electrodes. When the separated aqueous phase flows past the pH electrode during pH measurement, the electrodes are preferably arranged in a plane traverse to the direction of flow. Preferably, at least three pH electrodes are arranged side by side in the measuring section and the pH is determined as the average of the values measured by the pH electrodes, excluding the value that differs most from the other measured values. With these measures, malfunction of a pH electrode can be identified easily and reliably and has no influence on the determined pH. The use of several pH electrodes also allows calibration of pH electrodes without interruption of pH detection.

In the method of the invention, the determined pH is maintained in a predetermined range by adding acid or base to the loop reactor. Preferably, the pH is maintained at an essentially constant value. Preferably, a mixer or a circulation pump is arranged in the loop reactor between the point at which the acid or base is added and the measuring section to provide sufficient mixing between the reaction mixture and the added acid or base before determining the pH.

A buffer may be added to aid in maintaining the pH in the desired range. The buffer may be an inorganic buffer, such as a phosphate buffer, or preferably an organic buffer, such as a carboxylic acid / carboxylate buffer. The buffer may be prepared previous to feeding it to the loop reactor or may preferably be generated within the loop reactor by separately feeding the acid and the base which form the buffer into the loop reactor. When an epoxidation catalyst comprising a manganese complex is used, an oxalic acid / oxalate buffer is preferably used, which then acts both as buffer and as co-catalyst.

When an epoxidation catalyst comprising a manganese complex is used, the pH is preferably maintained in the range of from 2 to 6, more preferably 3 to 5. When the olefin is propene, the pH is preferably maintained in a range of from 3.5 to 4.8. When the olefin is allyl chloride, the pH is preferably maintained in a range of from 2.5 to 4.

When an epoxidation catalyst comprising a heteropolytungstate is used, the pH is preferably maintained in a range of from 1.5 to 4.

### Examples

### General

Continuous epoxidation of propene was carried out in a loop reactor constructed from steel tubes with a cooling mantle and static mixers arranged within the tubes. The loop reactor comprised in series feed lines for starting materials, two circulation pumps, and a withdrawal line for reaction mixture. The withdrawal line for reaction mixture was connected to phase separators for separating withdrawn reaction mixture into a liquid aqueous phase, a liquid organic phase and a gas phase. Nitrogen was introduced into the second phase separator and gas phase was withdrawn with a pressure regulating valve to maintain a constant pressure of from 1.45 to 1.50 MPa. The loop of the loop reactor had a total volume of 1200 ml and was operated at a circulation rate of 100 kg/h. The loop reactor comprised a measuring section with an enlarged cross section before the first circulation pump, through which reaction mixture passed in upward flow at a reduced flow rate, which caused a temporary phase separation in the measuring section. Three pH electrodes InPro® 4800/120/Pt100 from Mettler Toledo were arranged side by side in a part of the measuring section where aqueous phase separated from the flow. A fourth pH electrode of the same type was installed in a connecting tube of the loop reactor immediately before the withdrawal line for reaction mixture.

Epoxidations were carried out at 14 to 15 °C with separate feeding of 12 g/h of a 0.53 % by weight aqueous catalyst solution containing [(TmTacn)Mn^{IV}(µ-O)₃Mn^{IV}(TmTacn)](CH₃COO)₂ as catalyst, 393 g/h of an aqueous buffer solution containing 0.58 % by weight oxalic acid and 0.58 % by weight sodium oxalate, 165 g/h of a 60 % by weight aqueous hydrogen peroxide solution and 546 g/h of liquid propene.

### Example 1

In example 1, the pH of the aqueous phase of the reaction mixture was maintained at a value of 4.0, based on the average of the values measured by the pH electrodes in the measuring section, excluding the value that differs most from the other measured values, by adding small amounts of 10 % by weight aqueous sulfuric acid if needed. Fig.1 shows the pH values measured with pH electrodes I, II and III arranged in the measuring section and pH electrode IV arranged in the main flow of the loop reactor. Fig. 1 demonstrates that pH measurement without phase separation can lead to strong variations of the measured value that would severely affect pH control by addition of a base or an acid.

### Example 2

Example 1 was repeated, manually adjusting the amount of added sulfuric acid from time to time to maintain the target pH value. Fig.2 shows the pH values measured with pH electrodes I, II and III arranged in the measuring section and pH electrode IV arranged in the main flow of the loop reactor. Fig. 2 demonstrates that by using three pH electrodes and excluding the value that differs most from the other measured values for determining the pH value used for pH control allows to control the pH of the reaction mixture at the desired target value even when pH electrodes show substantial temporary measuring errors.

## Claims

1. A method for the epoxidation of an olefin, comprising continuously reacting the olefin with hydrogen peroxide in the presence of a homogeneous epoxidation catalyst, the reaction being carried out in a reaction mixture comprising an aqueous liquid phase and an organic liquid phase using a loop reactor with mixing of the liquid phases, **characterized in that** the loop reactor comprises a measuring section in which the liquid phases are temporarily separated into a separated aqueous phase and a separated organic phase, at least one pH electrode is arranged in said measuring section in contact with the separated aqueous phase, a pH of the separated aqueous phase is determined with said pH electrode and said pH is maintained in a predetermined range by adding acid or base to the loop reactor.

2. The method of claim 1, **characterized in that** the liquid phases are temporarily separated by lowering the flow rate.

3. The method of claim 2, **characterized in that** the flow rate is lowered in the measuring section by enlarging the flow cross section.

4. The method of any one of claims 1 to 3, **characterized in that** the measuring section is located in a side stream to the loop reactor.

5. The method of claim 4, **characterized in that** a valve is used for lowering the flow rate or temporarily stopping the flow in the measuring section.

6. The method of any one of claims 1 to 5, **characterized in that** at least three pH electrodes are arranged side by side in the measuring section.

7. The method of claim 6, **characterized in that** the pH is determined as the average of the values measured by the pH electrodes, excluding the value that differs most from the other measured values.

8. The method of any one of claims 1 to 7, **characterized in that** the epoxidation catalyst comprises a manganese complex carrying a 1,4,7-trimethyl-1,4,7-triazacyclonane ligand.

9. The method of claim 8, **characterized in that** the olefin is propene and the pH is maintained in a range of from 3.5 to 4.8.

10. The method of claim 8, **characterized in that** the olefin is allyl chloride and the pH is maintained in a range of from 2.5 to 4.

11. The method of any one of claims 1 to 7, **characterized in that** the epoxidation catalyst comprises a heteropolytungstate and the pH is maintained in a range of from 1.5 to 4.

## Patentansprüche

1. Verfahren zur Epoxidierung eines Olefins, umfassend die kontinuierliche Umsetzung des Olefins mit Wasserstoffperoxid in Gegenwart eines homogenen Epoxidierungskatalysators, wobei die Umsetzung in einer eine wässrige flüssige Phase und eine organische flüssige Phase umfassenden Reaktionsmischung unter Verwendung eines Schlaufenreaktors mit Mischen der flüssigen Phasen durchgeführt wird, **dadurch gekennzeichnet, dass** der Schlaufenreaktor einen Messabschnitt umfasst, in dem die flüssigen Phasen vorübergehend in eine getrennte wässrige Phase und eine getrennte organische Phase getrennt werden, in dem Messabschnitt mindestens eine pH-Elektrode in Kontakt mit der getrennten wässrigen Phase angeordnet ist, mit der pH-Elektrode ein pH-Wert der getrennten wässrigen Phase bestimmt wird und der pH-Wert durch Zugabe von Säure oder Base zum Schlaufenreaktor in einem vorbestimmten Bereich gehalten wird.

2. Verfahren nach Anspruch 1, **dadurch gekennzeichnet, dass** die flüssigen Phasen durch Verringerung der Strömungsrate vorübergehend getrennt werden.

3. Verfahren nach Anspruch 2, **dadurch gekennzeichnet, dass** die Strömungsrate im Messabschnitt durch Vergrößerung des Strömungsquerschnitts verringert wird.

4. Verfahren nach einem der Ansprüche 1 bis 3, **dadurch gekennzeichnet, dass** sich der Messabschnitt in einem Seitenstrom des Schlaufenreaktors befindet.

5. Verfahren nach Anspruch 4, **dadurch gekennzeichnet, dass** ein Ventil zum Verringern der Strömungsrate oder vorübergehenden Stoppen des Stroms im Messabschnitt verwendet wird.

6. Verfahren nach einem der Ansprüche 1 bis 5, **dadurch gekennzeichnet, dass** im Messabschnitt mindestens drei pH-Elektroden nebeneinander angeordnet sind.

7. Verfahren nach Anspruch 6, **dadurch gekennzeichnet, dass** der pH-Wert als Durchschnitt der von den pH-Elektroden gemessenen Werte unter Ausschluss desjenigen Werts, der von den anderen gemessenen Werten am meisten abweicht, bestimmt wird.

8. Verfahren nach einem der Ansprüche 1 bis 7, **dadurch gekennzeichnet, dass** der Epoxidierungskatalysator einen Mangankomplex, der einen 1,4,7-Trimethyl-1,4,7-triazacyclononan-Liganden trägt, umfasst.

9. Verfahren nach Anspruch 8, **dadurch gekennzeichnet, dass** es sich bei dem Olefin um Propen handelt und der pH-Wert in einem Bereich von 3,5 bis 4,8 gehalten wird.

10. Verfahren nach Anspruch 8, **dadurch gekennzeichnet, dass** es sich bei dem Olefin um Allylchlorid handelt und der pH-Wert in einem Bereich von 2,5 bis 4 gehalten wird.

11. Verfahren nach einem der Ansprüche 1 bis 7, **dadurch gekennzeichnet, dass** der Epoxidierungskatalysator ein Heteropolywolframat umfasst und der pH-Wert in einem Bereich von 1,5 bis 4 gehalten wird.

## Revendications

1. Procédé pour l'époxydation d'une oléfine, comprenant la réaction en continu de l'oléfine avec du peroxyde d'hydrogène en présence d'un catalyseur d'époxydation homogène, la réaction étant conduite dans un mélange de réaction comprenant une phase liquide aqueuse et une phase liquide organique au moyen d'un réacteur en boucle avec mélange des phases liquides, **caractérisé en ce que** le réacteur en boucle comprend une section de mesure dans laquelle les phases liquides sont temporairement séparées en une phase aqueuse séparée et une phase organique séparée, au moins une électrode de pH est agencée dans ladite section de mesure en contact avec la phase aqueuse séparée, un pH de la phase aqueuse séparée est déterminé avec ladite électrode de pH et ledit pH est maintenu dans une plage prédéterminée par ajout d'acide ou de base dans le réacteur en boucle.

2. Procédé selon la revendication 1, **caractérisé en ce que** les phases liquides sont temporairement séparées par diminution du débit.

3. Procédé selon la revendication 2, **caractérisé en ce que** le débit est diminué dans la section de mesure par agrandissement de la section transversale d'écoulement.

4. Procédé selon l'une quelconque des revendications 1 à 3, **caractérisé en ce que** la section de mesure est située dans un flux latéral du réacteur en boucle.

5. Procédé selon la revendication 4, **caractérisé en ce qu'**une vanne est utilisée pour diminuer le débit ou arrêter temporairement l'écoulement dans la section de mesure.

6. Procédé selon l'une quelconque des revendications 1 à 5, **caractérisé en ce qu'**au moins trois électrodes de pH sont agencées côte à côte dans la section de mesure.

7. Procédé selon la revendication 6, **caractérisé en ce que** le pH est déterminé comme étant la moyenne des valeurs mesurées par les électrodes de pH, en excluant la valeur qui diffère le plus des autres valeurs mesurées.

8. Procédé selon l'une quelconque des revendications 1 à 7, **caractérisé en ce que** le catalyseur d'époxydation comprend un complexe de manganèse comportant un ligand 1,4,7-triméthyl-1,4,7-triazacyclonane.

9. Procédé selon la revendication 8, **caractérisé en ce que** l'oléfine est le propène et le pH est maintenu dans une plage de 3,5 à 4,8.

10. Procédé selon la revendication 8, **caractérisé en ce que** l'oléfine est le chlorure d'allyle et le pH est maintenu dans une plage de 2,5 à 4.

11. Procédé selon l'une quelconque des revendications 1 à 7, **caractérisé en ce que** le catalyseur d'époxydation comprend un hétéropolytungstate et le pH est maintenu dans une plage de 1,5 à 4.
